# EUROPEAN PATENT SPECIFICATION

(11) **EP 1 106 593 B1**
(45) Date of publication and mention of the grant of the patent: **04.02.2004**
(21) Application number: 01106072.0
(22) Date of filing: 01.04.1998
(51) Int. Cl.: C07C 43/196, C09D 4/06

(54) **A composition comprising a vinyl reactive coalescent**
Zubereitung enthaltend ein vinyl-reaktives Fliesshilfsmittel
Composition contenant un agent coalescent réactif vinylique

(30) Priority: 08.04.1997 US 42725
(43) Date of publication of application: 13.06.2001
(62) Divisional of application: 98302549.5
(73) Proprietor: ROHM AND HAAS COMPANY, Philadelphia, Pennsylvania 19106-2399 (US)
(72) Inventor: Speece, David, Gerald, Jr., c/o Rohm and Haas Co., Philadelphia, PA 19106 (US); Eisenhart, Eric Karl, Ambler, PA 19002 (US); Bowe, Michael, Damian, Doylestown, PA 18901 (US); Weir, William, David, Levittown, PA 19056 (US); Wolfersberger, Martha, Alice, Harbaugh, Missouli, Montana 59803-2642 (US)
(74) Representative: Buckley, Guy Julian

(56) References cited:
- US-A- 4 131 580
- US-A- 4 141 868
- US-A- 4 145 503
- US-A- 4 540 738

## Description

This invention relates to a composition comprising a vinyl reactive coalescent

Water based polymers having a low glass transition temperature ("Tg") can be formulated into coatings without plasticizers. These coatings often have inadequate hardness for many applications. Many applications, such as gloss and semigloss paint formulations, require the properties of a hard polymer, i.e. a polymer with a Tg significantly above the ambient temperature. To meet these needs, a volatile coalescent or plasticizer is typically used to achieve film formation. The use of these volatile solvents in coatings is coming under scrutiny due to pollution and odor problems.

Attempts have been made to use "reactive coalescents". Reactive coalescents are compounds which aid in film formation similar to the conventional coalescent but are non-volatile and react to become part of the final coating.

U.S. Pat. No. 4,141,868 discloses the use of dicyclopentenyloxyethyl methacrylate ("DCPOEMA") as a reactive coalescent. Vinyl reactive coalescents are reactive coalescents which contain a vinyl group. DCPOEMA, dicyclopentenyloxyethyl acrylate ("DCPOEA"), dicyclopentenyloxy acrylate ("DCPOA"), and dicyclopentenyloxy methacrylate ("DCPOMA") are examples of vinyl reactive coalescents.

US-A-4 540 738 discloses the use of hydroquinone to prevent premature polymerisation. US-A-4 145 503 and US-A-4 131 580 disclose the addition of a ketone-oxime or an aldehyde-oxime as stabilizing agents in vinyl polymer containing compositions.

Despite the disclosure, there is a continuing need for stable reactive coalescents for use in coating compositions.

We have surprisingly found that stable reactive coalescents for use in coating compositions can be obtained by adding an inhibitor to the reactive coalescent.

The present invention provides a composition comprising:
from 1% to 25% of a vinyl reactive coalescent based on a weight reactive coalescent to total weight of composition basis;
from 1 ppm to 10,000 ppm of an inhibitor selected from the group consisting of 4-methoxyphenol, 2,2,6,6-tetramethyl-1-piperidinyloxy, 4-oxo-2,2,6,6-tetramethyl-1-piperidinyloxy, and di-tertiary butyl nitroxyl, based on a weight inhibitor to total weight of composition basis; and
from 74% to 99% of an emulsion polymer based on a weight emulsion polymer to total weight of composition basis.

The inhibitors useful in this invention are 4-methoxyphenol ("MEHQ"), 2,2,6,6-tetramethyl-1-piperidinyloxy ("4-HT"), 4-oxo-2,2,6,6-tetramethyl-1-piperidinyloxy (each available from Aldrich Chemical Company) and Di-tertiary butyl nitroxyl (available from Nova Molecular Technologies, Lake Geneva, Wisconsin). The inhibitors are typically used at levels of from 1 ppm to 10,000 ppm on a weight inhibitor to total weight of the composition basis. Preferred are inhibitor levels of from 100 ppm to 1,000 ppm. More preferred are inhibitor levels of from 200 ppm to 700 ppm.

The reactive coalescents useful in this invention are prepared by processes known in the art. For example, direct esterification, transesterification or the use of acid halides may be employed to convert alcohols to esters. Examples include, but are not limited to: dicyclopentenyloxyethyl acetate, dicyclopentenyloxyethyl butyrate, dicyclopentenyloxy-2-(2-hydroxyethoxy)ethane, dicyclopentenyloxy -2-(2-butoxyethoxy)ethane, dicyclopentenyloxyhexanol, dicyclopentenyloxyhexane, dicyclopentenyloxybutane, dicyclopentenyloxy (2-methyl)propane, and 1,2-bis (dicyclopentenyloxy)ethane.

The reactive coalescents are typically used at levels of from 1% to 25% on a weight reactive coalescent to total weight of composition basis. Preferred are reactive coalescent levels of from 2% to 20%. More preferred are reactive coalescent levels of from 5% to 15%.

This invention may be applied to any emulsion polymer. The preparation of emulsion polymers is known in the art, see for example U.S. Pat. No. 5,346,954.

The following is a list of products used within this invention and their sources:

| Product | Source |
|---|---|
| Tamol® 165 | Rohm and Haas Company |
| Triton® CF-10 | Union Carbide |
| Drew® ZV-22 | Ashland Chemical Company |
| RM-2020NPR | Rohm and Haas Company |
| Ti-Pure® R-706 | DuPont Chemical Company |
| Rhoplex® EXP-3361 | Rohm and Haas Company |
| Scotch® Tape | Minnesota Mining and Manufacturing Company |
| Tamol® 731 | Rohm and Haas Company |
| BYK-22 | BYK-Chemie GmbH |
| GR-7M | Union Carbide |
| Triton® XN-45S | Union Carbide |

The following examples are intended to demonstrate the compounds and compositions of this invention and their use as stable reactive coalescents.

### Example 1 - Improved Stability Of DCPOEMA

Reactive coalescents containing unsaturated vinyl groups can polymerize in the can due to residual impurities in the polymer. The poor stability of the coalescent manifests itself as loss of film forming properties of the formulated coating over time. DCPOEMA is a reactive coalescent which contains 100 ppm hydroquinone as purchased. Various inhibitor types and levels were added to DCPOEMA prior to use in a coating composition. The reactive coalescent and inhibitor mixtures were prepared prior to the compositions by adding DCPOEMA to a fixed amount of solid grade inhibitor. The mixtures were then post-added to a portion of a master mix of paint 1. A portion of each sample was stored at 60°C (140°F) for 10 days. The minimum film forming temperature ("MFFT") was measured for each sample. If the DCPOEMA was stable, the MFFT remained low when compared to the control sample. If the DCPOEMA was unstable, the MFFT was elevated when compared to the control. The DCPOEMA was considered to be stable if the MFFT increased less than 5°C after the sample was stored at 60°C (140°F) for 10 days. The results of the tests are shown in Table 1.

### Paint 1

A master paint formulation containing no coalescent was prepared as follows:
18 g water, 1.61 g Tamol®165, 0.50 g Triton®CF-10, 0.25 g Drew®ZV-22,
3.75 g RM-2020NPR, and 45.0 g Ti-Pure®R-706 were ground with a high speed disperser for 15 minutes. To the mixture was added 131.27 g EXP-3361,
0.25 g Drew®ZV-22, 1.18 g RM2020NPR, and 40.25 g water with further mixing.

Sample 1 was a control sample of Paint 1 with DCPOEMA, but no additional inhibitor.
Sample 2 was prepared by combining 7.55 g of a mixture of 0.29 g of solid grade 4-HT dissolved in 57.71 g of DCPOEMA with 244.06 g of paint 1.
Sample 3 was prepared by combining 7.55 g of a mixture of 0.60 g of solid grade 4-HT dissolved in 59.40 g of DCPOEMA with 244.06 g of paint 1.
Sample 4 was prepared by combining 7.55 g of a mixture of 1.20 g of solid grade 4-HT dissolved in 58.80 g of DCPOEMA with 244.06 g of paint 1.
Sample 5 was prepared by combining 7.55 g of a mixture of 2.37 g of solid grade 4-HT dissolved in 56.88 g of DCPOEMA with 244.06 g of paint 1.
Sample 6 was prepared by combining 7.55 g of a mixture of 0.30 g of MEHQ dissolved in 59.70 g of DCPOEMA with 244.06 g of paint 1.
Sample 7 was prepared by combining 7.55 g of a mixture of 0.60 g of MEHQ dissolved in 59.40 g of DCPOEMA with 244.06 g of paint 1.
Sample 8 was prepared by combining 7.55 g of a mixture of 1.16 g of MEHQ dissolved in 56.84 g of DCPOEMA with 244.06 g of paint 1.
Sample 9 was prepared by combining 7.55 g of a mixture of 2.42 g of MEHQ dissolved in 58.08 g of DCPOEMA with 244.06 g of paint 1.
Sample 10 (comparative) was prepared by combining 7.55 g of a mixture of 0.24 g of HQ dissolved in 57.71 g of DCPOEMA with 244.06 of paint 1.
Sample 11 (comparative) was prepared by combining 7.55 g of a mixture of 0.60 g of HQ dissolved in 59.40 g of DCPOEMA with 244.06 g of paint 1.
Sample 12 (comparative) was prepared by combining 7.55 g of a mixture of 1.19 g of HQ dissolved in 58.31 g of DCPOEMA with 244.06 g of paint 1.
Sample 13 (comparative) was prepared by combining 7.55 g of a mixture of 0.31 g of PTZ dissolved in 61.69 g of DCPOEMA with 244.06 g of paint 1.
Sample 14 (comparative) was prepared by combining 7.55 g of a mixture of 0.61 g of PTZ dissolved in 60.39 g of DCPOEMA with 244.06 g of paint 1.
Sample 15 (comparative) was prepared by combining 7.55 g of a mixture of 1.21 g of PTZ dissolved in 59.29 g of DCPOEMA with 244.06 of paint 1.

### The MFFT test was performed as follows:

Scotch®Tape biaxially oriented polypropylene was placed on an ICI Sheen MFFT Bar SS-3300. Samples were drawn down over the tape using a 1 inch cube Sheen Film Applicator with a gap size of 75 micron. After 60 to 90 minutes, the MFFT was read as the point where the film becomes continuous and not cracked.

### The Low Temperature Film Formation test was performed as follows:

Samples were painted by brush onto white pine boards. The paint was applied in strips perpendicular to the length of the board. Each sample was weighed to provide a spread rate of 41.8 m²/liter. The painted boards were dried for 24 hours at 4.5°C (40°F)/ 70% relative humidity. A magnifying glass was used to determine the degree of cracking in the paint film. The degree of cracking was reported according to the following scale:
10 = none, 9 = trace, 8 = trace/slight, 7 = slight, 6 = slight/moderate,
5 = moderate, 4 = moderate/heavy, 3 = heavy, 2 = heavy/very heavy,
1 = very heavy.

A score of 10 is considered to be passing.

**Table 1**

| | | Inhibitor | MFFT (°C) | |
|---|---|---|---|---|
| Sample | Inhibitor | Level | Initial | 10 Days@140F |
| 1 C | None | None | 5.1 | >18 |
| 2 | 4-HT | 625 ppm | 5.1 | 5.6 |
| 3 | 4-HT | 1250 ppm | 5.3 | 5.6 |
| 4 | 4-HT | 2500 ppm | 5.3 | 5.6 |
| 5 | 4-HT | 5000 ppm | 5.3 | 5.6 |
| 6 | MEHQ | 625 ppm | 4.9 | 5.6 |
| 7 | MEHQ | 1250 ppm | 4.9 | 6.4 |
| 8 | MEHQ | 2500 ppm | 4.9 | 7.6 |
| 9 | MEHQ | 5000 ppm | 4.5 | 7.6 |
| 10 C | HQ | 625 ppm | 7.8 | >18 |
| 11 C | HQ | 1250 ppm | 8.0 | >18 |
| 12 C | HQ | 2500 ppm | 9.2 | >18 |
| 13 C | PTZ | 625 ppm | 8.0 | >18 |
| 14 C | PTZ | 1250 ppm | 7.8 | >18 |
| 15 C | PTZ | 2500 ppm | 9.2 | >18 |

The results demonstrate that 4-HT and MEHQ improve the stability of DCPOEMA. HQ and PTZ did not improve the stability of DCPOEMA.

### Example 2 - The Level Of Inhibitor Required To Stabilze DCPOEMA

The level of 4-HT required to provide stability in commercial polymers was evaluated. Samples were prepared by adding 4-HT to latex, this was called a modified latex. The modified latex was then formulated into paints. Sub-samples of Samples 16 - 19 were stored at 60°C (140°F) for 30 days. Sub-samples of Samples 20 - 23 were stored at 60°C (140°F) for 10 days. The MFFT was measured for each Sample. The results are shown in Tables 2 and 3.

### Paint 2

Paint 2 was prepared for Samples 16 - 19 as follows:
72 g water, 12 g Tamol®731, 2 g Triton®CF-10, 2 g BYK 22,
20 g RM-2020NPR, and 300 g Ti-Pure®R-706 were ground with a high speed disperser for 15 minutes. To the mixture was added 106.32 g water, 2 g GR-7M,
544.14 g modified Rhoplex®AC-261, 26.84 g DCPOEMA, 2 g BYK-22, and 10 g water with further mixing.

### Paint 3

Paint 3 was prepared for Samples 20 - 23 as follows:
72 g water, 12 g Tamol®731, 2 g Triton®CF-10, 2 g BYK 22,
20 g RM-2020NPR, and 300 g Ti-Pure®R-706 were ground with a high speed disperser for 15 minutes. To the mixture was added 106.32 g water, 2 g GR-7M,
544.14 g modified Rhoplex®EXP-3361, 26.84 g DCPOEMA, 2 g BYK-22, and 10 g water with further mixing.

Sample 16 was a control paint 2 with DCPOEMA, but no additional inhibitor.
Sample 17 was prepared by combining 3.84 g of a 5% aqueous 4-HT mixture with 961.85 g of paint 2.
Sample 18 was prepared by combining 7.68 g of a 5% aqueous 4-HT mixture with 961.85 g of paint 2.
Sample 19 was prepared by combining 11.50 g of a 5% aqueous 4-HT mixture with 961.85 g of paint 2.
Sample 20 was prepared by combining 20.24 g of a 5% aqueous 4-HT mixture with 2,929,60 g of paint 3.
Sample 21 was prepared by combining 26.99 g of a 5% aqueous 4-HT mixture with 2,946,35 g of paint 3.
Sample 22 was prepared by combining 43.50 g of a 5% aqueous 4-HT mixture with 2,949.10 g of paint 3.
Sample 23 was prepared by combining 58.10 g of a 5% aqueous 4-HT mixture with 2,963.70 g of paint 3.

**Table 2**

| | | Low Temperature Film Formation | |
|---|---|---|---|
| Sample | 4-HT Level | Equilibrated | 30 Days@140F |
| 16 | 0 ppm | 10 | 1 |
| 17 | 400 ppm | 10 | 10 |
| 18 | 800 ppm | 10 | 10 |
| 19 | 1200 ppm | 10 | 10 |

**Table 3**

| | | MFFT | MFFT |
|---|---|---|---|
| Sample | 4-HT Level | Initial | 10 Day@140F |
| 20 | 750 ppm | 9.5°C | 9.5°C |
| 21 | 1000 ppm | 7.9°C | 10.2°C |
| 22 | 1600 ppm | 8.3°C | 10.9°C |
| 23 | 2100 ppm | 9.3°C | 10.9°C |

Tables 2 and 3 demonstrate that levels as low as 400ppm of 4-HT were effective at stabilizing the reactive coalescent.

## Claims

1. A composition comprising:
from 1% to 25% of a vinyl reactive coalescent based on a weight reactive coalescent to total weight of composition basis;
from 1 ppm to 10,000 ppm of an inhibitor selected from the group consisting of 4-methoxyphenol, 2,2,6,6-tetramethyl-1-piperidinyloxy, 4-oxo-2,2,6,6-tetramethyl-1-piperidinyloxy, and di-tertiary butyl nitroxyl, based on a weight inhibitor to total weight of composition basis; and
from 74% to 99% of an emulsion polymer based on a weight emulsion polymer to total weight of composition basis.

2. A composition according to claim 1 wherein the vinyl reactive coalescent is dicyclopentenyloxyethylmethacrylate and the inhibitor is 4-methoxyphenol.

## Patentansprüche

1. Zusammensetzung umfassend:
zwischen 1% und 25% eines Vinyl-reaktiven Koaleszenzmittels, bezogen auf das Verhältnis des Gewichts des reaktiven Koaleszenzmittels zum Gesamtgewicht der Zusammensetzung;
zwischen 1 ppm und 10.000 ppm eines Inhibitors, ausgewählt aus der Gruppe bestehend aus 4-Methoxyphenol, 2,2,6,6-Tetramethyl-1-piperidinyloxy, 4-Oxo-2,2,6,6-Tetramethyl-1-piperidinyloxy, und Di-tertiärbutylnitroxyl, bezogen auf das Verhältnis des Gewichts des Inhibitors zum Gesamtgewicht der Zusammensetzung; und
zwischen 74% und 99% eines Emulsionspolymers, bezogen auf das Verhältnis des Gewichts des Emulsionspolymers zum Gesamtgewicht der Zusammensetzung.

2. Zusammensetzung nach Anspruch 1, wobei das Vinyl-reaktive Koaleszenzmittel Dicyclopentenyloxyethylmethacrylat und der Inhibitor 4-Methoxyphenol ist.

## Revendications

1. Composition comprenant :
1 % à 25 %, par rapport au poids total de la base de la composition, d'un agent coalescent réactif vinylique à base d'un agent coalescent réactif, en poids ;
1 ppm à 10 000 ppm, par rapport au poids total de la base de la composition, d'un inhibiteur choisi dans le groupe constitué par le 4-méthoxyphénol, le 2,2,6,6-tétraméthyl-1-pipéridinyloxy, le 4-oxo-2,2,6,6-tétraméthyl-1-pipéridinyloxy, et le di-butyle tertiaire-nitroxyle, sur la base d'un inhibiteur, en poids ; et
74 % à 99 %, par rapport au poids total de la base de la composition, d'un polymère en émulsion sur la base d'un polymère en émulsion, en poids.

2. Composition suivant la revendication 1, dans laquelle l'agent coalescent réactif vinylique est le méthacrylate de dicyclopentényloxyéthyle, et l'inhibiteur est le 4-méthoxyphénol.
